# EUROPEAN PATENT SPECIFICATION

(11) **EP 0 595 807 B1**
(45) Date of publication and mention of the grant of the patent: **05.03.1997**
(21) Application number: 91914310.7
(22) Date of filing: 23.07.1991
(51) Int. Cl.: A61M 5/158, A61M 39/02

(54) **A CLOSURE FOR A MEDICAMENT WELL**
VERSCHLUSS FÜR MEDIKAMENTENRESERVOIR
BOUCHON DE FERMETURE POUR RESERVOIR DE MEDICAMENT

(43) Date of publication of application: 11.05.1994
(73) Proprietor: PREISS, Otto T., 0393 Oslo 3 (NO); Heimreid, Bent, N-3942 Skjelsvik (NO)
(72) Inventor: HEIMREID, Bent, N-3942 Skjelsvik (NO)
(74) Representative: Stolt, Lars C.
(86) International application number: PCT/NO91/00103
(87) International publication number: WO 93/01848

(56) References cited:
- EP-A- 0 309 771
- US-A- 1 166 338
- US-A- 1 267 616
- US-A- 4 666 427

## Description

The present invention relates to a closure for a medicament well.

More precisely, the invention relates to a closure in combination with a medicament well on a cannula, the closure being removable and comprising a central, closing body.

There is mainly one kind of intravenous cannulae on the market at present, viz. a kind where a circular medicament well projecting normally to the liquid channel in the intravenous cannula is closed from above by the aid of a conventional cap, which is commonly fastened to the sleeve of the medicament well by the aid of a more or less sturdy fastening strap.

The present cannulae with a medicament well of a conventional kind show the inherent deficiency that after administration of medicament and removal of the administrating syringe, there is always a small volume of medicament solution left in the well due to the counterpressure from the liquid flowing in the cannula.

This phenomenon may represent quite a health hazard, since the volume of medicament remaining in the medicament well may act as a substrate for bacteria, or the medicament may be subjected to decomposition or metamorphosis due to influence from the outside environment.

This is probably one of the reasons for the fact that this kind of intravenous cannula is not accepted by the FDA on the American market.

Body heat may also cause undesirable changes.

Often, hours may pass between each time the medicament well is used, and this enhances the hazard of undesirable changes.

When the medicament well is used again, the medicament phase which may be modified or decomposed will be pushed into the cannula and introduced into the body, which may cause undesirable and even dangerous states.

Another aspect that should be considered in this connection is that when small volumes of medicaments are administered it may occur that not all of the medicament is injected, since somewhat is left in the well.

US-A-4,666,427 describes a fluid- and particle-absorbing body for apertures in injection instruments. However, this device represents a health risk and a safety problem in the health care of today. US-A-1,267,616 describes an urethritic syringe representing an old prior art not able to solve the present problems.

It is an object of the present invention to remedy the shortcomings of known technology and to provide a device which permits complete discharge of the well. In this manner any hazard of administrating contaminated material to the patient is avoided, decomposition or modification of a medicament remaining in the well is avoided, and complete administration of the medicament to the patient is ensured.

This is achieved by the aid of a closure of the above mentioned kind, and this closure is characterized by a void provided in the upper end of the closure, which is defined against the outside on top by a flexible wall and is on the other side made to communicate with the outside in the well, via a channel extending through the closure body, the volume of the void substantially being equal to the residue of medicament solution which is seen to remain at the bottom of the well.

The invention is disclosed in more detail with reference to the accompanying drawings, in which
- Figure 1: shows a medicament well with an inserted syringe for administration of a medicament;
- Figure 2: shows a conventional closure;
- Figure 3: shows a closure according to the invention in the same phase as the conventional closure according Figure 2; and
- Figure 4: shows a closure according to the invention immediately after the remaining residue was squeezed out of the well.

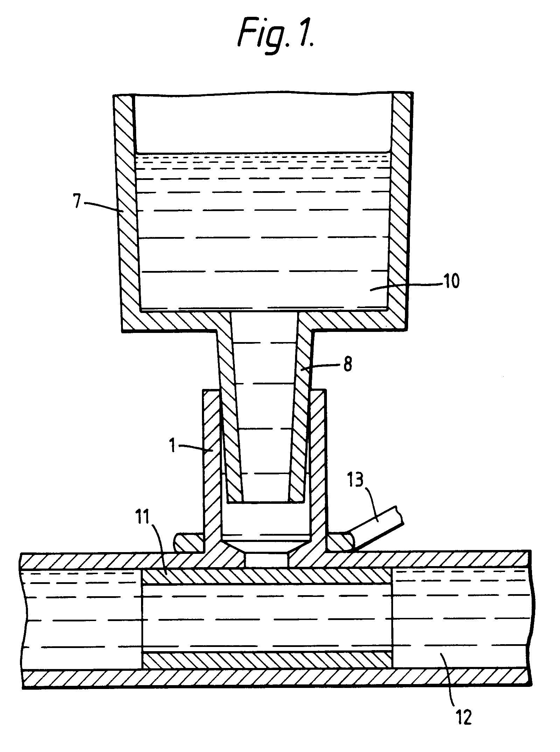

In Figure 1 a syringe 7 with a luer 8 is inserted into well 1 for administration of medicament solution 10.

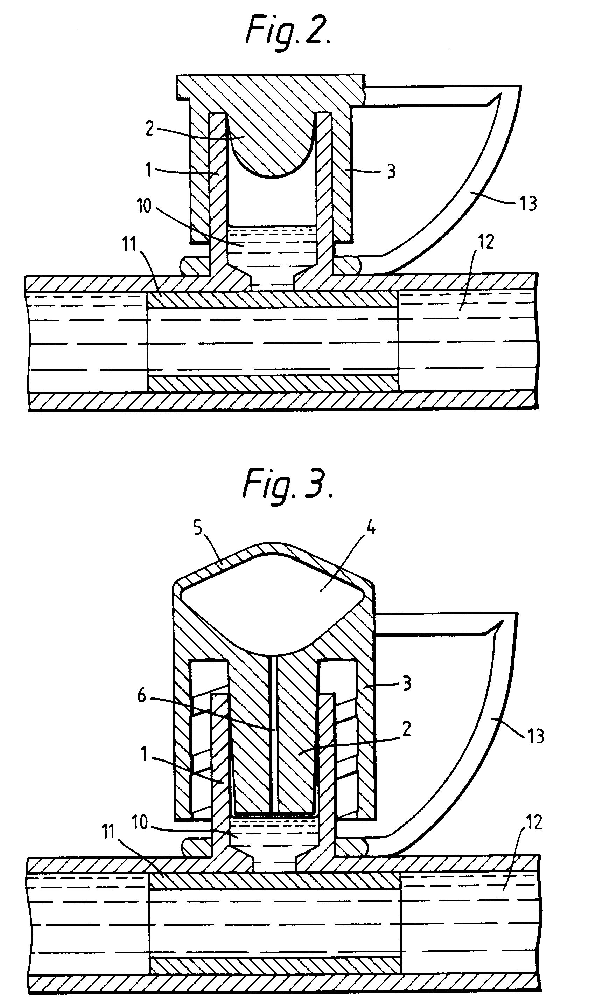

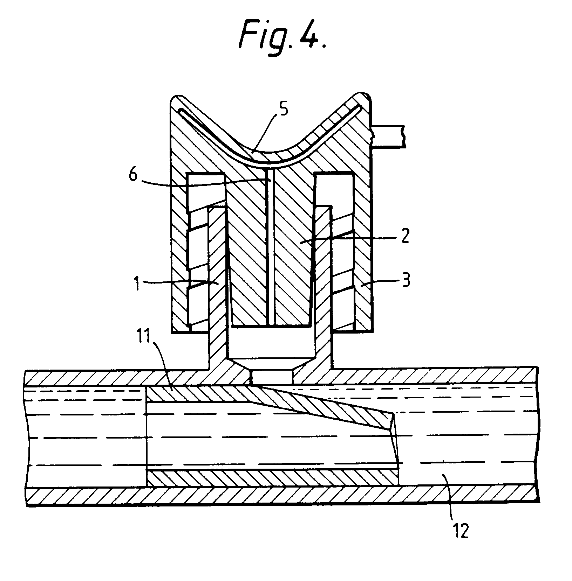

When medicament solution 10 is injected into the liquid path 12 inside the cannula, valve 11 is urged to open, as indicated in Figure 4. Upon completed injection the counter-pressure in liquid 12 will urge valve 11 back into contact with the cannula wall, and upon removal of syringe 10 and insertion of a closure the situation is as shown in Figures 2 and 3, with a conventional closure, and a closure according to the invention, respectively.

As indicated, a residue of medicament solution 10 will remain at the bottom of well 1, and this residue may, as mentioned above, be subjected to decomposition or metamorphosis, and/or it may represent great values of economic and therapeutic kind.

As mentioned above, it is an object of the invention to permit utilization of said residue 10 as well by removing it from well 1 and urge it into the liquid path 12.

This is achieved by the aid of the arrangement according to the invention by the fact that the flexible wall 5 which defines an air-filled void 4 uppermost in the closure is compressed, as shown in Figure 4. Consequently, air is discharged through channel 6 in the central body 2 of the closure and will urge residual liquid 10 past valve 11 and into the liquid path 12.

If desired, a closure skirt 3 may extent around the body 2, substantially in parallel with the body.

Upon compression of void 4 and removal of residual liquid 10 the counterpressure in liquid 12 will cause valve 11 to contact the cannula wall.

According to the invention the volume of the air void 4 is substantially equal to the volume of the residue of medicament solution 10 remaining at the bottom of well 1 for complete removal of said residue without air being introduced into the liquid flow 12.

It is, thus, possible to remove remaining medicament solution from wells or corresponding injection sleeves in a simple and hygienic manner in order to avoid the above indicated problems in a simple and safe manner.

## Claims

1. A closure in combination with a medicament well (1) on a cannula, the closure being removable and comprising a central closing body (2), characterized in that a void (4) is provided at the upper end of the closure, the void (4) being defined towards the outside on the upper side by a flexible wall (5) and on the other side made to communicate with the outside in the well (1) via a channel (6) extending through the closing body (2), the volume of the void (4) substantially being equal to the residue of medicament solution (10) which is seen to remain at the bottom of the well (1).

2. A closure according to claim 1, characterized by a closure skirt (3) extending around the body (2) of the closure substantially in parallel with the body (2).

## Patentansprüche

1. Verschluß in Kombination mit einem Medikamentenreservoir (1) auf einer Kanüle, welcher Verschluß abnehmbar ist und einen zentralen Verschlußkörper (2) umfaßt, dadurch gekennzeichnet, daß am oberen Ende des Verschlusses ein Hohlraum (4) vorgesehen ist, der nach außen hin an der Oberseite durch eine flexible Wand (5) begrenzt ist und auf der anderen Seite so ausgebildet ist, daß er mit der Außenseite des Reservoirs (1) über einen sich durch den Verschlußkörper (2) erstreckenden Kanal (6) in Verbindung steht, wobei das Volumen des Hohlraumes (4) im wesentlichen dem Rest der Medikamentenlösung (10) entspricht, die am Boden des Reservoirs (1) sichtbar bleibt.

2. Verschluß nach Patentanspruch 1, gekennzeichnet durch einen Verschlußmantel (3), der sich um den Körper (2) des Verschlusses im wesentlichen parallel zum Körper 2 erstreckt.

## Revendications

1. Fermeture en combinaison avec une alvéole à médicament (1) sur une canule, la fermeture pouvant être retirée et comprenant un corps fermant central (2), caractérisée en ce qu'un vide (4) est prévu à l'extrémité supérieure de la fermeture, le vide (4) étant défini en direction de l'extérieur du côté supérieur par une paroi flexible (5) et d'autre part mis en communication avec l'extérieur dans l'alvéole (1) par un canal (6) s'étendant à travers le corps de fermeture (2), le volume du vide (4) étant sensiblement égal au résidu de solution médicamenteuse (10) que l'on voit rester au fond de l'alvéole (1).

2. Fermeture selon la revendication 1, caractérisée par une jupe de fermeture (3) s'étendant autour du corps (2) de la fermeture sensiblement en parallèle avec le corps (2).
